# EUROPEAN PATENT SPECIFICATION

(11) **EP 0 357 238 B1**
(45) Date of publication and mention of the grant of the patent: **07.07.1999**
(21) Application number: 89307889.9
(22) Date of filing: 03.08.1989
(51) Int. Cl.: A01N 59/00, A01N 37/16, A01N 31/02

(54) **Anti-microbial composition**
Antimikrobielle Zusammensetzung
Composition antimicrobienne

(30) Priority: 08.08.1988 US 229917
(43) Date of publication of application: 07.03.1990
(60) Divisional application: 98105838.1
(73) Proprietor: STERIS CORPORATION, Mentor, Ohio 44060 (US)
(72) Inventor: Kralovic, Raymond C., Austinburg Ohio (US); Badertscher, Duncan C., Cleveland Ohio (US)
(74) Representative: Parr, Ronald Edward

(56) References cited:
- US-A- 4 518 585
- CHEMICAL ABSTRACTS, vol. 83, no. 8, 25th August 1975, page 385, abstract no. 64532w, Columbus, Ohio, US; H. MUECKE: "Attempts to reduce peracetic acid corrosion of iron, copper, brass, and bronze using phosphate", & PHARMAZIE 1975, 30(4), 238-40
- CHEMICAL ABSTRACTS, vol. 93, no. 6, 11th August 1980, page 353, abstract no. 52264d, Columbus Ohio, US; E. MUECKE: "Further studies on the protection of metals against the corrosive action of diluted peracetic acid by the addition of certain phosphates" & PHARMAZIE 1979, 34(9), 573
- DERWENT JAPANESE PATENTS GAZETTE, section Ch, week Y46, 3rd January 1978, Derwent Publications Ltd, London, GB; & JP-A-52 120 118 (TOKYO SHIBAURA ELEC. LTD) 08-10-1977

## Description

The present invention relates to antimicrobial compositions and methods of using them. It finds particular application in conjunction with automated sterilizing or disinfecting of medical instruments and will be described with particular reference thereto. However, it is to be appreciated that the present invention will find utility in sterilizing and disinfecting a wide range of objects, either automatically or manually.

Heretofore, most medical instruments have been sterilized in a steam autoclave. In hospitals and large facilities, medical instruments and equipment were transported to a central sterilizing facility where they were sterilized under the supervision of sterilizing room technicians. In a steam autoclave, the equipment was subject to superheated steam at high pressures, depressurized, and cooled. One of the drawbacks of the steam autoclave is that many medical instruments cannot withstand the high temperatures and pressures. Another drawback resides in the one to two hour cycle time.

Instruments and equipment which could not withstand the pressure or temperature of the autoclave were commonly sterilized with ethylene oxide gas. The equipment was sealed in a sterilizing chamber which was pressurized with the ethylene oxide gas. After an appropriate sterilizing cycle, the equipment was degassed for twelve to sixteen hours in a vacuum or about 72 hours in ambient atmospheric conditions to remove the highly toxic ethylene oxide. One of the drawbacks to ethylene oxide sterilization resided in the long cycle times. Another drawback resided in the need for training technicians to handle the highly toxic ethylene oxide gas systems. Yet another drawback was that some medical equipment could not be sterilized with ethylene oxide gas.

Liquid sterilization systems were utilized for equipment which could not withstand either the autoclave or the ethylene oxide. The equipment was immersed in a vat or tank that had been filled with a sterilizing solution, such as stabilized hydrogen peroxide or glutaraldehyde. Because such liquid sterilizations were normally performed manually, the skill and care of the technician were controlling factors in whether sterilization or disinfection were, in fact, attained. In many instances, the technician was required to mix the components of the anti-microbial composition. Even when mixed properly, relatively long immersion times on the order of six to ten hours were commonly required to assure sterilization. Moreover, many liquid sterilization systems were highly corrosive to metal parts, particularly brass, copper, and aluminum. With long immersion times, even carbon steel and stainless steel could be pitted and sharp cutting edges dulled.

Chemical Abstracts, vol. 93, no. 6, 11th August 1980, page 353, abstract no. 52264d discloses a means of protecting from corrosion iron, bronze, and brass in peracetic acid solutions by the use of Na₂H₂P₂O₇ or a 1:1 mixture of that compound with NaH₂PO₄H₂O.

Chemical Abstracts, vol. 83, no. 8, 25 August 1975, page 385, abstract no. 64532w discloses a means of reducing the corrosive effect of peracetic acid solutions on copper, brass and bronze by the addition of NaH₂PO₄.

Derwent Japanese Patents Gazette, section Ch, week Y46, 3rd January 1978 describes a means of preventing corrosion by ozone solution in metal water pipes, using sodium hexametaphosphate and potassium bichromate.

US-A-4 518 585 (D.F. Greene et al) discloses aqueous acidic disinfecting and sterilizing compositions containing hydrogen peroxide, a surfactant and a triazole corrosion inhibitor.

In accordance with the present invention, a new and improved anti-microbial composition is provided which overcomes the above-mentioned problems.

In a first aspect the invention provides an anti-microbial composition characterised in that it comprises:
(a) a strong oxidant selected from peracetic acid and lithium hypochlorite;
(b) a copper and brass corrosion inhibitor selected from five-membered ring compounds and benzoates;
(c) a buffering agent which buffers the composition to a pH in the range from pH 6.4 to pH 7.9;
(d) an anti-corrosive agent which inhibits corrosion of carbon steel, stainless steel and aluminum;
(e) a wetting agent.

In a second aspect the present invention provides an anti-microbial composition, characterised in that it comprises:
(i) a strong oxidant selected from peracetic acid and lithium hypochlorite;
(ii) an azole as an inhibitor of copper and brass corrosion; and
(iii)a phosphate as a buffering agent which buffers the composition to a pH in the range from pH 6.4 to pH 7.9, and as an anti-corrosive agent.

A third aspect of the present invention provides a method of sterilising or disinfecting, characterised in that a surface to be sterilised or disinfected is brought into contact with an anti-microbial composition according to said first or second aspect of the invention.

Where the strong oxidant is lithium hypochlorite the composition is conveniently buffered to a pH in the range 6.95 to 7.9, for example in the range 6.95 to 7.0. Where the strong oxidant is peracetic acid the composition is conveniently buffered to a pH of 6.4.

The anti-microbial composition, whether according to the first or second aspect of the invention, is conveniently in the form of an aqueous solution.

In accordance with another aspect of the present invention, an anti-microbial solution is provided which comprises peracetic acid or lithium hypochlorite, a copper and brass corrosion inhibitor, an aluminum corrosion inhibitor, a steel corrosion inhibitor and buffering agent, and a wetting agent. If tap water is used as a dilutant it may be convenient - though not essential - to use a sequestering agent. Where the strong oxidant is peracetic acid it is preferably in an amount in the range 0.005 - 1.0%, for example 0.2%. Where the strong oxidant is lithium hypochlorite it is conveniently used in an amount of 0.05 to 0.13%.

In accordance with another more limited aspect of the invention, the copper and brass corrosion inhibitor can comprise a triazole or other azole. Where it is a triazole it can be particularly benzotriazole or tolyltriazole.

In accordance with another more limited aspect of the invention, the aluminum and steel corrosion inhibitor and the buffering agent are selected from the class consisting of chromates, dichromates, borates, phosphates, molybdates, vanadates, and tungstates. More specifically to a preferred embodiment, phosphates are preferred for inhibiting steel corrosion and buffering the solution to a substantial pH. Molybdates are preferred for inhibiting aluminum corrosion.

As explained below, the use of a wetting agent is advantageous. However, excellent results can be obtained using compositions of the invention without a wetting agent. This is clearly illustrated by Example 1 below where excellent results were obtained on a range of metals using a peracetic acid composition containing no wetting agent.

In accordance with a more limited aspect of the invention, a method of sterilizing medical instruments is provided. Powdered water soluble reagents including a copper and brass corrosion inhibitor, an anti-corrosive buffering agent, a sequestering agent, and a wetting agent are dissolved in water and circulated through the plumbing of an automated sterilizing apparatus and over the medical instruments to be sterilized. A concentrated, peracetic acid or lithium hypochlorite agent is diluted into the water with dissolved powder reagents and circulated through the plumbing of the automated apparatus and over the medical instruments. After the diluted antimicrobial has remained in contact with the medical instrument for a selected duration, the anti-microbial solution is drained and the medical instrument is rinsed with sterilized water.

In accordance with a more limited aspect of the sterilizing method, the reagents are selected from the classes discussed above.

One advantage of the present invention is that it provides an antimicrobial agent which quickly sterilizes or disinfects medical equipment or the like.

Another advantage of the present invention is that it works on substantially all materials with minimal corrosion.

Yet another advantage of the present invention is that it facilitates automated sterilizing and minimizes operator error.

Still further advantages of the present invention will become apparent upon reading and understanding the following detailed description.

The invention may utilize various components and arrangements of components or in various steps and arrangements of steps. The drawings are only for purposes of illustrating a preferred embodiment and are not to be construed as limiting the invention.
FIGURE 1 is a perspective view of a sterilizing apparatus in accordance with the present invention; and,
FIGURE 2 is a tubing diagram of the sterilizer of FIGURE 1.

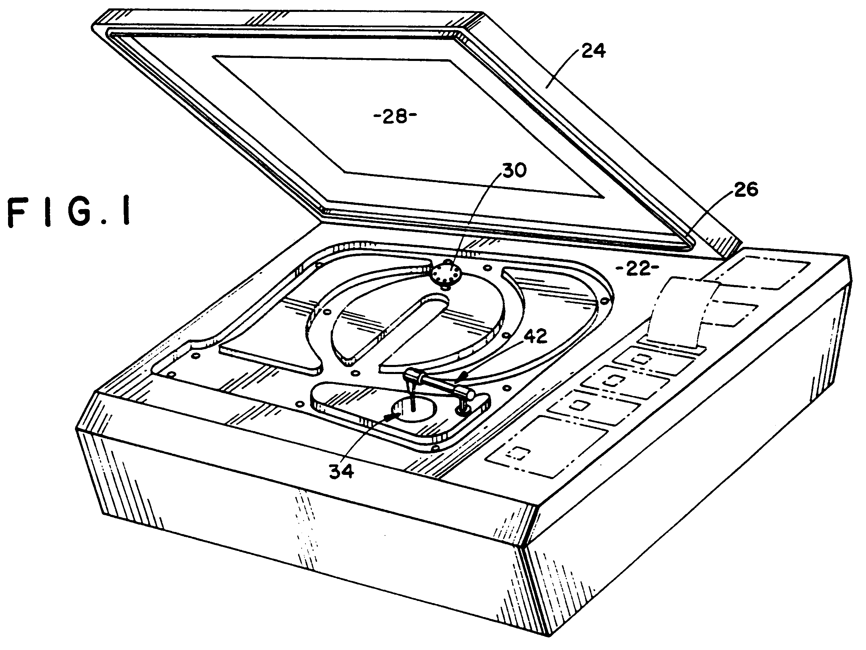

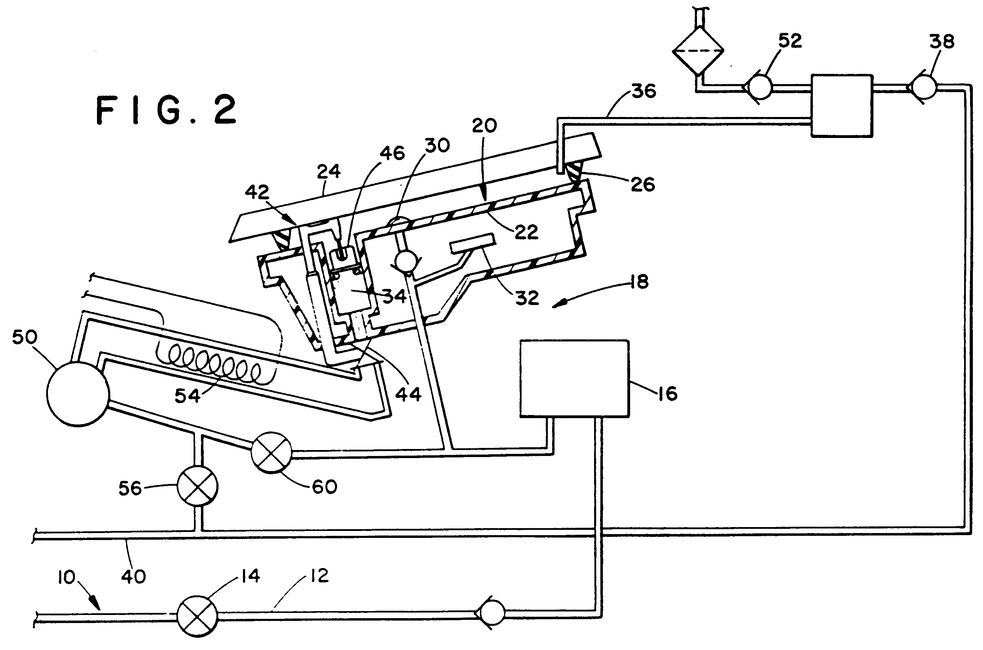

With reference to FIGURES 1 and 2, a dilutant or water source **10** supplies water or other fluid reagents. In the preferred sterilizer embodiment, the water source includes a length of tubing **12** connected with a water spigot or other building plumbing and a control valve **14** for selectively preventing and permitting the flow of water to a sterilizing means **16**. In the preferred embodiment, the sterilizing means is a filter which removes particles which are as large or larger than bacteria. Optionally, an inline water treatment means may be provided for modifying the chemical composition of the water. For example, a water softening cartridge may be provided for reducing or eliminating calcium and magnesium salts from the water. Alternately, various water treatments may be added to the water, such as a wetting agent, a sequestering agent, or others of the reagents to be discussed herein below.

A tubing system **18** connects the filter or sterilizing means with a container or module **20** for receiving an item to be sterilized. In the preferred embodiment, the container is defined by a removable tray **22** configured in accordance with the item, e.g. an endoscope. A lid **24** is sealed to the tray in a lowered position by a resilient gasket **26** to complete the container. Optionally, a transparent window **28** is defined in the lid.

The tubing system includes a spray nozzle **30** and a container distribution manifold **32** for distributing water and other fluids around the item to be sterilized. A reagent receiving well **34** collects the fluid for return. Vent lines **36** enable air to be vented from the container such that it is completely filled with the water or other sterilant solution. Any excess fluid is discharged through check valve **38** into a drain line **40**.

A reagent introduction system **42** introduces corrosion inhibitors and peracetic acid or lithium hypochlorite anti-microbial agent into the water. Preferably, the corrosion inhibitors are introduced first and circulated over the item to be sterilized or disinfected before the anti-microbial agent. This provides corrosion protection before the corrosive anti-microbial agent contacts the item. Alternately, the corrosion inhibitors and anti-microbial agent may be introduced concurrently such that both reach the item contemporaneously. An aspirator **44** which draws a oxidizing sterilant concentrate or other liquids from an ampule **46**. In a preferred embodiment, the oxidant is concentrated peracetic acid which has a stable shelf life of several months. More specific to the preferred embodiment, the operator opens a container having a premeasured dose of powdered reagents and the liquid filled ampule. The powdered reagents are emptied into the well **34** and the ampules positioned to be pierced and drained by the aspirator. As described in greater detail below, the powdered reagents include a buffer, e.g. a phosphate, for bringing the pH to a neutral level and to inhibit steel corrosion.

An aluminum corrosion inhibitor, such as a molybdate inhibits aluminum and steel corrosion. A copper and brass corrosion inhibitor, preferably a triazole, protects copper and brass components. Moreover, there is a synergistic interaction in which the phosphates, molybdates, triazoles, and wetting agent interact to protect steel, aluminum, copper, and brass better than if any one were eliminated. If tap water, rather than deionized water is used as the dilutant, a sequestering agent, e.g. sodium hexametaphosphate, is included to prevent the precipitation of its calcium and magnesium salts.

After placement of the item the operator closes the lid and the system is filled with water. A pump **50** selectively draws water from the container **20** through well **34** and aspirator **44**. Recirculating the water dissolves the powdered reagents and aspirates the sterilant from the ampule and circulates the sterilant and reagents through the tubing system **18**. Preferably, the vent line **36** is very short and of a substantial diameter such that the solution is circulated over exposed surfaces of the drain check valve **38** and a vent check valve **52**. A heating coil adjusts the temperature of the solution. Recirculation continues until the interior of the container and all exposed surfaces of the tubing system and valves are sterilized. Alternately, once fully dissolved, the sterilant may remain quiescent for a selected duration.

After the preselected sterilization or disinfecting period, the sterilant solution is drained through a drain valve **56** and sterile air is drawn into the system through an air sterilizing means - preferably a filter that removes any particles the size of a bacteria or larger. The filled valve **14** is opened and the drain valve **56** is closed such that the sterile filter **16** provides a source of sterile rinse. Note that the sterile rinse passes only along sterilized surfaces of the tubing system and valves in order to assure sterility. Every tubing and valve surface from the filter to the drain was exposed to the circulating sterilant solution for a sufficient duration to assure its sterility. The pump **50** circulates the sterile rinse through the system for a selected duration sufficient to rinse residue that the buffered strong oxidant tends to deposit. At the end of the rinse cycle, the rinse solution is drained by opening drain valve **56**. When a return valve **60** is closed, the pump **50** functions to pump liquid from the system out the drain **40**. Additional drain lines (not shown) and aspirators or pumps (not shown) may be provided for removing liquids from every region of the system. The exact location of such additional drains will be dependent on the bends and contours of the plumbing system.

In this preferred embodiment, the ampule contains 35% peracetic acid and appropriate stabilizers, as are known in the art to provide an acceptable shelf life. The volume of the ampule relative to the volume of the tubing system **18** is selected such that the peracetic acid is diluted to about 0.2% by weight per volume solution. However, final solutions of .005 - 1% may prove acceptable. The anti-microbial agent and its concentration, and the contact time with the item will vary depending on whether disinfection or sterilization is sought. In many applications, only disinfection is sought. The contact time or the strength composition of the anti-microbial agent may be adjusted accordingly.

The copper and brass corrosion inhibitors are preferably benzotriazoles and tolyltriazoles, which are preferred due to their stability in the presence of strong oxidizing compounds. Mercaptobenzathiozol might also be utilized but is more apt to be oxidized or destabilized by strong oxidizers.

Azoles, benzoates, and other five membered ring compounds may also prove acceptable as copper and brass corrosion inhibitors.

The anti-corrosive buffering compounds are preferably a mixture of phosphate in sufficient volume to produce a final concentration of 1.25% weight per volume and molybdates in an appropriate amount to produce a final solution of .011% weight per volume. Phosphates may also be effective in the range of 0.2% to 12.5% and the molybdates may be effective from 0.1 to 10%. Optionally, borates, chromates, dichromates, tungstates, vanadates and combinations thereof, may be substituted, in appropriate concentrations to inhibit steel corrosion, i.e. buffer to a generally neutral pH, and aluminum corrosion.

In hard water, the phosphates tend to cause calcium and magnesium salts to precipitate and coat the instruments being sterilized and parts of the sterilizing system. A sequestering agent appropriate to prevent precipitation, such as sodium hexametaphosphate, may be provided. Of course, if deionized or soft water is utilized the sequestering agent may be eliminated. However, to insure universal applicability with any water that might be utilized, the presence of a sequestering agent is preferred.

A wetting agent present to about 0.001 percent (W/V) improves the wetting of the surface of the instrument by the anti-microbial agent. The wetting agent has also been found to increase penetration by the anti-microbial agent improving anti-microbial efficacy while reducing corrosion.

The following are examples that illustrate the corrosion inhibiting effectiveness of a peracetic acid anti-microbial composition of the invention (Example 1) compared with peracetic acid alone (Example 2), and of several lithium hypochlorite anti-microbial compositions (Examples 3 to 7) of the invention compared with lithium hypochlorite alone (Examples 8 and 9). Particular attention was paid to corrosion of the metals:

### Metals

A Brass
B Stainless Steel
C Aluminum
D Carbon Steel
E Copper

In the following Examples, the degree of corrosion has been assessed on the following scale:
1. No corrosion;
2. Slight corrosion;
3. Moderate corrosion;
4. High corrosion; and
5. Heavy corrosion.

### Example 1

| Formula 2000 ppm Peracetic Acid 0.125% Monosodium Phosphate 1.12% Disodium Phosphate 0.08% 1-H-Benzotriazole 0.025% Sodium Hexametaphosphate 0.05% Sodium Molybdate pH 6.4 | | | | | |
|---|---|---|---|---|---|
| Exposure Time | Metal | | | | |
| | A | B | C | D | E |
| 30 minutes | 1 | 1 | 1 | 1 | 1 |
| 16 hours | 1 | 1 | 1 | 1 | 1 |
| 24 hours | 1 | 1 | 1 | 1 | |

### Example 2

| Formula 2000 ppm Peracetic Acid pH 2.0 | | | | | |
|---|---|---|---|---|---|
| Exposure Time | A | B | C | D | E |
| 1 Hour | 5 | 3 | 5 | 5 | |

### Example 3

| 1000 ppm Lithium Hypochlorite 0.125% Monosodium Phosphate 0.8% 1-H-Benzotriazole 0.025% Sodium Hexametaphosphate pH 7.9 | | | | | |
|---|---|---|---|---|---|
| Exposure Time | A | B | C | D | E |
| 30 minutes | 1 | 1 | 1 | 1 | |
| 90 minutes | 1 | 1 | 1 | 1 | |
| 65 hours | | 2 | 1 | 2 | 1 |

### Example 4

| 1300 ppm Lithium Hypochlorite 0.6% Disodium Phosphate 0.52% Monosodium Phosphate 0.09% Sodium Molybdate 0.07% 1-H-Benzotriazole 0.045% Sodium Hexametaphosphate 0.009% C0-720* pH 7.0 | | | | | |
|---|---|---|---|---|---|
| Exposure Time | A | B | C | D | E |
| 30 minutes | 1 | 1 | 1 | 1 | |
| 1 hour | 1 | 1 | 1 | 1 | |

### Example 5

| 500 ppm Lithium Hypochlorite 1.25% Monosodium Phosphate 0.5% Sodium Molybdate 0.03% Sodium Hexametaphosphate 0.05% CO-720* 0.08% 1-H-Benzotriazole pH 6.95 | | | | | |
|---|---|---|---|---|---|
| Exposure Time | A | B | C | D | E |
| 30 minutes | 1 | 1 | 1 | 1 | |

### Example 6

| 500 ppm Lithium Hypochlorite 1.25% Monosodium Phosphate 1.25% Disodium Phosphate 0.5% Sodium Molybdate 0.05% Sodium Hexametaphosphate 0.08% 1-H Benzotriazole 0.3% 2A1** pH 6.95 | | | | | |
|---|---|---|---|---|---|
| Exposure Time | A | B | C | D | E |
| 30 minutes | 1 | 1 | 1 | 1 | |

### Example 7

| 1000 ppm Lithium Hypochlorite 1.25% Monosodium Phosphate 1.25% Disodium Phosphate 1.0% Sodium Molybdate 0.08% 1-H-Benzotriazole pH 7.0 | | | | | |
|---|---|---|---|---|---|
| Exposure Time | A | B | C | D | E |
| 30 minutes | 1 | 1 | 1 | 1 | |
| 24 hours | 1 | 1 | 1 | 1 | |

### Example 8

| 1000 ppm Lithium Hypochlorite pH 11.2 | | | | | |
|---|---|---|---|---|---|
| Exposure Time | A | B | C | D | E |
| 1 hour | 3 | 3 | 5 | 5 | |

### Example 9

| 500 ppm Lithium Hypochlorite pH 11.0 | | | | | |
|---|---|---|---|---|---|
| Exposure Time | A | B | C | D | E |
| 1 hour | 3 | 3 | 5 | 5 | |

### Wetting Agents

* "CO-720": stands for a wetting agent sold by Rhone-Poulenc under the trade mark "Igepal CO-720". Igepal CO-720 = Poly(oxy-1,2-ethanediyl),alpha-(nonyphenyl)-omega-hydroxy-(C₂H₄O)ₙC₁₅H₂₄O n = 12
** A1 = Dodecyl (Sulphenoxy)benzene sulfonic acid, disodium salt and oxybis (dodecyl benzene sulfonic acid), disodium salt

## Claims (Claims for the following Contracting State(s): AT, BE, CH, DE, FR, GB, GR, IT, IL, LU, NL, SE)

1. An anti-microbial composition characterised in that it comprises:
(a) a strong oxidant selected from peracetic acid and lithium hypochlorite;
(b) a copper and brass corrosion inhibitor selected from five-membered ring compounds and benzoates;
(c) a buffering agent which buffers the composition to a pH in the range from pH 6.4 to pH 7.9;
(d) an anti-corrosive agent which inhibits corrosion of carbon steel, stainless steel and aluminum;
(e) a wetting agent.

2. An anti- microbial composition according to Claim 1, wherein said copper and brass corrosion inhibitor comprises a triazole or other azole.

3. An anti-microbial composition according to Claim 2, wherein said copper and brass corrosion inhibitor is selected from benzotriazoles and tolyltriazoles.

4. An anti-microbial composition according to any of the preceding claims, wherein the buffering agent is selected from chromates, dichromates, borates, phosphates, molybdates, vanadates and tungstates.

5. An anti-microbial composition according to Claim 4, wherein the buffering agent is a phosphate.

6. An anti-microbial composition according to any of the preceding claims, wherein said anti-corrosive agent is selected from chromates, dichromates, borates, phosphates, molybdates, vanadates and tungstates.

7. An anti-microbial composition according to Claim 6, wherein said anti-corrosive agent is a phosphate.

8. An anti-microbial composition according to Claim 5, wherein said phosphate constitutes said anti-corrosive agent and the buffering agent.

9. An anti-microbial composition according to any of the preceding claims, wherein said anti-corrosive agent includes a phosphate and at least one compound selected from molybdates, chromates, tungstates, borates and vanadates.

10. An anti-microbial composition according to any of the preceding claims, further including a sequestering agent to prevent any phosphate present in the composition from causing precipitation in hard water.

11. An anti-microbial composition according to any of the preceding claims, wherein said copper and brass corrosion inhibitor includes a triazole, and said anti-corrosive agent includes at least two compounds selected from chromates, dichromates, borates, phosphates, molybdates, vanadates and tungstates.

12. An anti-microbial composition characterised in that it comprises:
(i) a strong oxidant selected from peracetic acid and lithium hypochlorite;
(ii) an azole as an inhibitor of copper and brass corrosion; and
(iii)a phosphate as a buffering agent which buffers the composition to a pH in the range from pH 6.4 to pH 7.9, and as an anti-corrosive agent.

13. An antimicrobial composition according to Claim 1 or Claim 12, wherein the strong oxidant is peracetic acid and the composition is buffered to a pH of 6.4.

14. An anti-microbial composition according to Claim 1 or Claim 12, wherein the strong oxidant is lithium hypochlorite and the composition is buffered to a pH in the range 6.95 to 7.9.

15. An anti-microbial composition according to Claim 14, wherein the strong oxidant is lithium hypochlorite and the composition is buffered to a pH in the range 6.95 to 7.0.

16. An anti-microbial composition, according to Claim 12, which includes a wetting agent.

17. An anti-microbial composition according to any of Claims 12 to 16, wherein the azole is a benzo- or tolyl-triazole.

18. An anti-microbial composition according to Claim 17, wherein the azole is 1-H-benzotriazole.

19. An anti-microbial composition according to Claim 12, which also contains a sequestering agent to prevent phosphate present in the composition from causing precipitation in hard water.

20. An anti-microbial composition according to Claim 1, the composition comprising:
0.005-1% peracetic acid;
0.001-1% triazole;
0.2 - 12.5% phosphate;
0.01 to 10% of a compound selected from chromates, dichromates, borates, phosphates, molybdates, vanadates and tungstates;
0 - 0.01% of a sequestering agent; and
a wetting agent.

21. An anti-microbial composition according to any of the preceding claims, which is in the form of an aqueous solution.

22. A method of sterilising or disinfecting, characterised in that a surface to be sterilised or disinfected is brought into contact with an anti-microbial composition as claimed in any of Claims 1 to 21.

## Claims (Claims for the following Contracting State(s): ES)

1. A process for the production of an anti-microbial composition, characterised in that it comprises mixing together:
(a) a strong oxidant selected from peracetic acid and lithium hypochlorite;
(b) a copper and brass corrosion inhibitor selected from five-membered ring compounds and benzoates;
(c) a buffering agent which buffers the composition to a pH in the range from pH 6.4 to pH 7.9;
(d) an anti-corrosive agent which inhibits corrosion of carbon steel, stainless steel and aluminum;
(e) a wetting agent.

2. A process according to Claim 1, wherein said copper and brass corrosion inhibitor comprises a triazole or other azole.

3. A process according to Claim 2, wherein said copper and brass corrosion inhibitor is selected from benzotriazoles and tolyltriazoles.

4. A process according to any of the preceding claims, wherein the buffering agent is selected from chromates, dichromates, borates, phosphates, molybdates, vanadates and tungstates.

5. A process according to Claim 4 wherein the buffering agent is a phosphate.

6. A process according to any of the preceding claims, wherein said anti-corrosive agent is selected from chromates, dichromates, borates, phosphates, molybdates, vanadates and tungstates.

7. A process according to Claim 6, wherein said anti-corrosive agent is a phosphate.

8. A process according to Claim 5, wherein said phosphate constitutes said anti-corrosive agent and the buffering agent.

9. A process according to any of the preceding claims, wherein said anti-corrosive agent includes a phosphate and at least one compound selected from molybdates, chromates, tungstates, borates and vanadates.

10. A process according to any of the preceding claims, further including a sequestering agent to prevent any phosphate present in the composition from causing precipitation in hard water.

11. A process according to any of the preceding claims, wherein said copper and brass corrosion inhibitor includes a triazole, and said anti-corrosive agent includes at least two compounds selected from chromates, dichromates, borates, phosphates, molybdates, vanadates and tungstates.

12. A process for the production of an anti-microbial composition, characterised in that it comprises mixing together:
(i) a strong oxidant selected from peracetic acid and lithium hypochlorite;
(ii) an azole as an inhibitor of copper and brass corrosion; and
(iii)a phosphate as a buffering agent which buffers the composition to a pH in the range from pH 6.4 to pH 7.9, and as an anti-corrosive agent.

13. A process according to Claim 1 or Claim 12, wherein the strong oxidant is peracetic acid and the composition is buffered to a pH of 6.4.

14. A process according to Claim 1 or Claim 12, wherein the strong oxidant is lithium hypochlorite and the composition is buffered to a pH in the range 6.95 to 7.9.

15. A process according to Claim 14, wherein the strong oxidant is lithium hypochlorite and the composition is buffered to a pH in the range 6.95 to 7.0.

16. A process according to Claim 12, which includes a wetting agent.

17. A process according to any of Claim 12 to 16, wherein the azole is a benzo- or tolyl-triazole.

18. A process according to Claim 17, wherein the azole is 1-H-benzotriazole.

19. A process according to Claim 12, which also contains a sequestering agent to prevent phosphate present in the composition from causing precipitation in hard water.

20. A process according to Claim 1, the composition comprising:
0.005-10% peracetic acid;
0.001-1% triazole;
0.2 - 12.5% phosphate;
0.01 to 10% of a compound selected from chromates, dichromates, borates, phosphates, molybdates, vanadates and tungstates;
0 - 0.01% of a sequestering agent; and
a wetting agent.

21. A process according to any of the preceding claims, in which said composition is in the form of an aqueous solution.

22. A method of sterilising or disinfecting, characterised in that a surface to be sterilised or disinfected is brought into contact with an anti-microbial composition which has been produced by the process as claimed in any of Claims 1 to 21.

## Patentansprüche (Patentansprüche für folgende(n) Vertragsstaat(en): AT, BE, CH, DE, FR, GB, GR, IT, LI, LU, NL, SE)

1. Anti-mikrobielle Zusammensetzung, dadurch gekennzeichnet, dass sie:
(a) einen aus Peressigsäure und Lithiumhypochlorit ausgewählten starken Oxidator;
(b) einen aus fünfgliedrigen Ringverbindungen und Benzoaten ausgewählten Kupfer- und Messingkorrosionshemmstoff;
(c) ein Puffermedium, welches die Zusammensetzung auf einen pH-Wert in dem Bereich von 6,4 bis 7,9 puffert;
(d) ein anti-korrosives Medium, welches Korrosion von Kohlenstoffstahl, rostfreiem Stahl und Aluminium verhindert
enthält.

2. Anti-mikrobielle Zusammensetzung nach Anspruch 1, bei welcher der Kupfer- und Messingkorrosionshemmstoff ein Triazol oder andere Azole enthält.

3. Anti-mikrobielle Zusammensetzung nach Anspruch 2, bei welcher der Kupfer- und Messingkorrosionshemmstoff aus Benzotriazolen und Tolyltriazolen ausgewählt ist.

4. Anti-mikrobielle Zusammensetzung nach einem der vorhergehenden Ansprüche, bei welcher das Puffermdium aus Chromaten, Dichromaten, Boraten, Phosphaten, Molybdaten, Vanadaten und Wolframaten ausgewählt ist.

5. Anti-mikrobielle Zusammensetzung nach Anspruch 4, bei welcher das Puffermedium ein Phosphat ist.

6. Anti-mikrobielle Zusammensetzung nach einem der vorhergehenden Ansprüche, bei welcher das anti-korrosive Medium aus Chromaten, Dichromaten, Boraten, Phosphaten, Molybdaten, Vanadaten und Wolframaten ausgewählt ist.

7. Anti-mikrobielle Zusammensetzung nach Anspruch 6, bei welcher das anti-korrosive Medium ein Phosphat ist.

8. Anti-mikrobielle Zusammensetzung nach Anspruch 5, bei welcher das Phosphat das anti-korrosive Medium und das Puffermedium darstellt.

9. Anti-mikrobielle Zusammensetzung nach einem der vorhergehenden Ansprüche, bei welcher das anti-korrosive Medium ein Phosphat und mindestens eine Komponente aus Molybdaten, Chromaten, Wolframaten, Boraten und Vanadaten enthält.

10. Anti-mikrobielle Zusammensetzung nach einem der vorhergehenden Ansprüche, weiterhin enthaltend ein Trennmedium, um zu verhindern, dass ein sich in der Zusammensetzung befindliches Phosphat in hartem Wasser Niederschlag verursacht.

11. Anti-mikrobielle Zusammensetzung nach einem der vorhergehenden Ansprüche, bei welcher der Kupfer- und Messingkorrosionshemmstoff ein Triazol enthält, und bei welcher das anti-korrosive Medium mindestens zwei Komponenten aus der Gruppe Chromate, Dichromate, Borate, Phosphate, Molybdate, Vanadate und Wolframate enthält.

12. Anti-mikrobielle Zusammensetzung, dadurch gekennzeichnet, dass sie
(i) einen aus Peressigsäure und Lithiumhypochlorit ausgewählten starken Oxidator;
(ii) ein Azol als Hemmstoff für Kupfer- und Messingkorrosion; und
(iii) ein Phosphat als Puffermedium, welches die Zusammensetzung auf einen pH-Wert im Bereich von 6,4 bis 7,9 puffert, und als anti-korrosives Medium
enthält.

13. Anti-mikrobielle Zusammensetzung nach Anspruch 1 oder Anspruch 12, bei welcher der starke Oxidator Peressigsäure ist und die Zusammensetzung auf einen pH-Wert von 6,4 gepuffert wird.

14. Anti-mikrobielle Zusammensetzung nach Anspruch 1 oder Anspruch 12, bei welcher der starke Oxidator Lithiumhypochlorit ist und die Zusammensetzung auf einen pH-Wert im Bereich von 6,95 bis 7,9 gepuffert wird.

15. Anti-mikrobielle Zusammensetzung nach Anspruch 14, bei welcher der starke Oxidator Lithiumhypochlorit ist und die Zusammensetzung auf einen pH-Wert im Bereich von 6,95 bos 7,0 gepuffert wird.

16. Anti-mikrobielle Zusammensetzung nach Anspruch 12, welche ein Netzmittel enthält.

17. Anti-mikrobielle Zusammensetzung nach einem der Ansprüche 12 bis 16, bei welcher das Azol ein Benzo- oder Tolyltriazol ist.

18. Anti-mikrobielle Zusammensetzung nach Anspruch 17, bei welcher das Azol 1-H-Benzotriazol ist.

19. Anti-mikrobielle Zusammensetzung nach Anspruch 12, welche auch ein Trennmedium enthält, um zu verhindern, dass ein sich in der Zusammensetzung befindliches Phosphat in hartem Wasser Niederschlag verursacht.

20. Anti-mikrobielle Zusammensetzung nach Anspruch 1, enthaltend:
0,005 - 1% Peressigsäure;
0,001 - 1% Triazol;
0,2 - 12,5% Phosphat;
0,01 - 10% einer aus Chromaten, Dichromaten, Boraten, Phosphaten, Molybdaten, Vanadaten und Wolframaten ausgewählten Zusammensetzung;
0 - 0,01% Trennmedium; und
ein Netzmittel.

21. Anti-mikrobielle Zusammensetzung nach einem der vorhergehenden Ansprüche, welche in Gestalt einer wässrigen Lösung vorliegt.

22. Verfahren zum Sterilisieren oder Desinfizieren, dadurch gekennzeichnet, dass eine zu sterilisierende oder zu desinfizierende Oberfläche in Berührung mit einer anti-mikrobiellen Zusammensetzung nach einem der Ansprüche 1 bis 21 gebracht wird.

## Patentansprüche (Patentansprüche für folgende(n) Vertragsstaat(en): ES)

1. Verfahren zur Herstellung einer anti-mikrobiellen Zusammensetzung, dadurch gekennzeichnet, dass es ein Zusammenwirken von
(a) einem aus Peressigsäure und Lithiumhypochlorit ausgewählten starken Oxidator;
(b) einem aus fünfgliedrigen Ringverbindungen und Benzoaten ausgewählten Kupfer- und Messingkorrosionshemmstoff;
(c) einem Puffermdium, welches die Zusammensetzung auf einen pH-Wert im Bereich von 6,4 bis 7,9 puffert;
(d) einem anti-korrosives Medium, welches Korrosion von Kohlenstoffstahl, rostfreiem Stahl und Aluminium verhindert;
(e) einem Netzmittel
aufweist.

2. Verfahren nach Anspruch 1, bei welchem der Kupfer- und Messingkorrosionshemmstoff ein Triazol oder andere Azole enthält.

3. Verfahren nach Anspruch 2, bei welchem der Kupfer- und Messingkorrosionshemmstoff aus Benzotriazolen und Tolyltriazolen ausgewählt ist.

4. Verfahren nach einem der vorhergehenden Ansprüche, bei welchem das Puffermdium aus Chromaten, Dichromaten, Boraten, Phosphaten, Molybdaten, Vanadaten und Wolframaten ausgewählt ist.

5. Verfahren nach Anspruch 4, bei welchem das Puffermedium ein Phosphat ist.

6. Verfahren nach einem der vorhergehenden Ansprüche, bei welchem das anti-korrosive Medium aus Chromaten, Dichromaten, Boraten, Phosphaten, Molybdaten, Vanadaten und Wolframaten ausgewählt ist.

7. Verfahren nach Anspruch 6, bei welchem das anti-korrosive Medium ein Phosphat ist.

8. Verfahren nach Anspruch 5, bei welchem das Phosphat das anti-korrosive Medium und das Puffermedium darstellt.

9. Verfahren nach einem der vorhergehenden Ansprüche, bei welchem das anti-korrosive Medium ein Phosphat und mindestens eine Komponente aus Molybdaten, Chromaten, Wolframaten, Boraten und Vanadaten enthält.

10. Verfahren nach einem der vorhergehenden Ansprüche, weiterhin enthaltend ein Trennmedium, um zu verhindern, dass ein sich in der Zusammensetzung befindliches Phosphat in hartem Wasser Niederschlag verursacht.

11. Verfahren nach einem der vorhergehenden Ansprüche, bei welcher der Kupfer- und Messingkorrosionshemmstoff ein Triazol enthält, und bei welcher das anti-korrosive Medium mindestens zwei Komponenten aus der Gruppe Chromate, Dichromate, Borate, Phosphate, Molybdate, Vanadate und Wolframate enthält.

12. Verfahren zur Herstellung einer anti-mikrobiellen Zusammensetzung, dadurch gekennzeichnet, dass es ein Zusammen mischen von:
(i) einem aus Peressigsäure und Lithiumhypochlorit ausgewählten starken Oxidator;
(ii) einem Azol als Hemmstoff für Kupfer- und Messingkorrosion; und
(iii) einem Phosphat als Puffermedium, welches die Zusammensetzung auf einen pH-Wert im Bereich von 6,4 bis 7,9 puffert und als anti-korrosives Medium,
aufweist.

13. Verfahren nach Anspruch 1 oder Anspruch 12, bei welchem der starke Oxidator Peressigsäure ist und die Zusammensetzung auf einen pH-Wert von 6,4 gepuffert wird.

14. Verfahren nach Anspruch 1 oder Anspruch 12, bei welchem der starke Oxidator Lithiumhypochlorit ist und die Zusammensetzung auf einen pH-Wert im Bereich von 6,95 bis 7,9 gepuffert wird.

15. Verfahren nach Anspruch 14, bei welchem der starke Oxidator Lithiumhypochlorit ist und die Zusammensetzung auf einen pH-Wert im Bereich von 6,95 bos 7,0 gepuffert wird.

16. Verfahren nach Anspruch 12, welches ein Netzmittel enthält.

17. Verfahren nach einem der Ansprüche 12 bis 16, bei welchem das Azol ein Benzo- oder Tolyltriazol ist.

18. Verfahren nach Anspruch 17, bei welchem das Azol 1-H-Benzotriazol ist.

19. Verfahren nach Anspruch 12, welches auch ein Trennmedium enthält, um zu verhindern, dass ein sich in der Zusammensetzung befindliches Phosphat in hartem Wasser Niederschlag verursacht.

20. Verfahren nach Anspruch 1, bei welchem die Zusammensetzung
0,005 - 1% Peressigsäure;
0,001 - 1% Triazol;
0,2 - 12,5% Phosphat;
0,01 - 10% einer aus Chromaten, Dichromaten, Boraten, Phosphaten, Molybdaten, Vanadaten und Wolframaten ausgewählten Zusammensetzung;
0 - 0,01% Sequestermedium; und
ein Benetzungsmittel
enthält.

21. Verfahren nach einem der vorhergehenden Ansprüche, bei welchem die Zusammensetzung in Gestalt einer wässrigen Lösung vorliegt.

22. Verfahren zum Sterilisieren oder Desinfizieren, dadurch gekennzeichnet, dass eine zu sterilisierende oder zu desinfizierende Oberfläche in Berührung mit einer anti-mikrobiellen Zusammensetzung kommt, welche durch das in einem der Ansprüche 1 bis 21 beanspruchte Verfahren hergestellt worden ist.

## Revendications (Revendications pour l'(les) Etat(s) contractant(s) suivant(s): AT, BE, CH, DE, FR, GB, GR, IT, LI, LU, NL, SE)

1. Composition antimicrobienne caractérisée en ce qu'elle comprend :
(a) un oxydant puissant choisi parmi l'acide peracétique et l'hypochlorite de lithium;
(b) un inhibiteur de corrosion du cuivre et du laiton choisi parmi les composés cycliques à cinq chaînons et les benzoates;
(c) un agent tampon qui tamponne la composition à un pH compris dans la plage de pH 6,4 à pH 7,9;
(d) un agent anticorrosion qui inhibe la corrosion de l'acier au carbone, l'acier inoxydable et l,aluminium ;
(e) un agent mouillant .

2. Composition antimicrobienne selon la revendication 1, dans laquelle ledit inhibiteur de corrosion du cuivre et du laiton comprend un triazole ou un autre azole.

3. Composition antimicrobienne selon la revendication 2, dans laquelle ledit inhibiteur de corrosion du cuivre et du laiton est choisi parmi les benzotriazoles et tolyltriazoles.

4. Composition antimicrobienne selon l'une quelconque des revendications précédentes, dans laquelle l'agent tampon est choisi parmi les chromates, les bichromates, les borates, les phosphates, les molybdates, les vanadates et les tungstates.

5. Composition antimicrobienne selon la revendication 4, dans laquelle l'agent tampon est un phosphate.

6. Composition antimicrobienne selon l'une quelconque des revendications précédentes, dans laquelle ledit agent anticorrosion est choisi parmi les chromates, les bichromates, les borates, les phosphates, les molybdates, les vanadates et les tungstates.

7. Composition antimicrobienne selon la revendication 6, dans laquelle ledit agent anticorrosion est un phosphate.

8. Composition antimicrobienne selon la revendication 5, dans laquelle ledit phosphate constitue ledit agent anticorrosion et ledit agent tampon.

9. Composition antimicrobienne selon l'une quelconque des revendications précédentes, dans laquelle ledit agent anticorrosion comprend un phosphate et au mains un composé choisi parmi les molybdates, les chromates, les tungstates, les borates et les vanadates.

10. Composition antimicrobienne selon l'une quelconque des revendications précédentes, comprenant en outre un agent complexant afin d'empêcher que tout phosphate dans la composition ne provoque une précipitation dans l'eau dure.

11. Composition antimicrobienne selon l'une quelconque des revendications précédentes, dans laquelle ledit inhibiteur de corrosion du cuivre et du laiton comprend un triazole, et ledit agent anticorrosion comprend au moins deux composés choisis parmi les chromates, les bichromates, les borates, les phosphates, les molybdates, les vanadates et les tungstates.

12. Composition antimicrobienne caractérisée en ce qu'elle comprend :
(i) un oxydant puissant choisi parmi l'acide peracétique et l'hypochlorite de lithium;
(ii) un azole comme inhibiteur de corrosion du cuivre et du laiton; et
(iii) un phosphate comme agent tampon qui tamponne la composition à un pH compris dans la plage de pH 6,4 à pH 7,9, et comme agent anticorrosion.

13. Composition antimicrobienne selon la revendication 1 ou la revendication 12, dans laquelle l'oxydant puissant est l'acide peracétique et la composition est tamponnée à pH 6,4.

14. Composition antimicrobienne selon la revendication 1 ou la revendication 12, dans laquelle l'oxydant puissant est l'hypochlorite de lithium et la composition est tamponnée à un pH compris dans la plage de 6,95 à 7,9.

15. Composition antimicrobienne selon la revendication 14, dans laquelle l'oxydant puissant est l'hypochlorite de lithium et la composition est tamponnée à un pH compris dans la plage de 6,95 à 7,0.

16. Composition antimicrobienne, selon la revendication 12, qui comprend un agent mouillant.

17. Composition antimicrobienne selon l'une quelconque des revendications 12 à 16, dans laquelle l'azole est un benzo- ou tolyltriazole.

18. Composition antimicrobienne selon la revendication 17, dans laquelle l'azole est le 1-H-benzotriazole.

19. Composition antimicrobienne selon la revendication 12, qui contient également un agent complexant pour empêcher que le phosphate présent dans la composition ne provoque une précipitation dans l'eau dure.

20. Composition antimicrobienne selon la revendication 1, la composition comprenant :
de 0,005 à 1% d'acide peracétique
de 0,001% à 1% de triazole;
de 0,2 à 12,5% de phosphate;
de 0,01 à 10% d'un composé choisi parmi les chromates, les bichromates, les borates, les phosphates, les molybdates, les vanadates et les tungstates.
de 0 à 0,01% d'agent complexant; et
un agent mouillant.

21. Composition antimicrobienne selon l'une quelconque des revendications précédentes, qui se présente sous forme d'une solution aqueuse.

22. Procédé de stérilisation ou de désinfection, caractérise en ce qu'une surface devant être stérilisée ou désinfectée est mise en contact avec une composition antimicrobienne selon l'une quelconque des revendications 1 à 21.

## Revendications (Revendications pour l'(les) Etat(s) contractant(s) suivant(s): ES)

1. Procédé de production d'une composition antimicrobienne caractérisé en cc qu'il comprend le mélange :
(a) d'un oxydant puissant choisi parmi l'acide peracétique et l'hypochlorite de lithium;
(b) d'un inhibiteur de corrosion du cuivre et du laiton choisi parmi les composés cycliques à cinq chaînons et les benzoates;
(c) d'un agent tampon qui tamponne la composition à pH compris dans la plage de pH 6,4 à pH 7,9;
(d) d'un agent anticorrosion qui inhibe la corrosion de l'acier au carbone, de l'acier inoxydable et de l'aluminium;
(e) d'un agent mouillant.

2. Procédé selon la revendication 1, dans lequel ledit inhibiteur de corrosion du cuivre et du laiton comprend un triazole ou un autre azole.

3. Procédé selon la revendication 2, dans lequel ledit inhibiteur de corrosion du cuivre et du laiton est choisi parmi les benzotriazoles et tolyltriazoles.

4. Procédé selon l'une quelconque des revendications précédentes, dans lequel l'agent tampon est choisi parmi les chromates, les bichromates, les borates, les phosphates, les molybdates, les vanadates et les tungstates.

5. Procédé selon la revendication 4, dans lequel l'agent tampon est un phosphate.

6. Procédé selon l'une quelconque des revendications précédentes, dans lequel ledit agent anticorrosion est choisi parmi les chromates, les bichromates, les borates, les phosphates, les molybdates, les vanadates et les tungstates.

7. Procédé selon la revendication 6, dans lequel ledit agent anticorrosion est un phosphate.

8. Procédé selon la revendication 5, dans lequel ledit phosphate constitue ledit agent anticorrosion et l'agent tampon.

9. Procédé selon l'une quelconque des revendications précédentes, dans lequel ledit agent anticorrosion comprend un phosphate et au moins un composé choisi parmi les molybdates, les chromates, les tungstates, les borates et les vanadates.

10. Procédé selon l'une quelconque des revendications précédentes, comprenant en outre l'addition d'un agent complexant afin d'empêcher tout phosphate présent dans la composition de provoquer une précipitation dans l'eau dure.

11. Procédé selon l'une quelconque des revendications précédentes, dans lequel ledit inhibiteur de corrosion du cuivre et du laiton comprend un triazole, et ledit agent anticorrosion comprend au moins deux composés choisis parmi les chromates, les bichromates, les borates, les phosphates, les molybdates, les vanadates et les tungstates.

12. Procédé de production d'une composition antimicrobienne caractérisé en ce qu'il comprend le mélange :
(i) d'un oxydant puissant choisi parmi l'acide peracétique et l'hypochlorite de lithium;
(ii) d'un azole comme inhibiteur de corrosion du cuivre et du laiton; et
(iii) d'un phosphate comme agent tampon qui tamponne la composition à pH compris dans la plage de pH 6,4 à pH 7,9, et comme un agent anticorrosion.

13. Procédé selon la revendication 1 ou la revendication 12, dans lequel l'oxydant puissant est l'acide peracétique et la composition est tamponnée à pH 6,4.

14. Procédé selon la revendication 1 ou la revendication 12, dans lequel l'oxydant puissant est l'hypochlorite de lithium et la composition est tamponnée à un pH compris dans la plage de 6,95 à 7,9.

15. Procédé selon la revendication 14, dans lequel l'oxydant puissant est l'hypochlorite de lithium et la composition est tamponnée à un pH compris dans la plage de 6,95 à 7,0.

16. Procédé selon la revendication 12, qui comprend l'addition d'un agent mouillant.

17. Procédé selon l'une quelconque des revendications 12 à 16, dans lequel l'azole est un benzo- ou tolyltriazole.

18. Procédé selon la revendication 17, dans lequel l'azole est le 1-H-benzotriazole.

19. Procédé de production d'une composition antimicrobienne selon la revendication 12, qui contient également un agent complexant pour empêcher que le phosphate présent dans la composition ne provoque une précipitation dans l'eau dure.

20. Procédé selon la revendication 1, la composition comprenant :
de 0,005 à 1% d'acide peracétique
de 0,001% à 1% de dérivé triazole;
de 0,2 à 12,5% de phosphate;
de 0,01 à 10% d'un composé choisi parmi les chromates, les bichromates, les borates, les phosphates, les molybdates, les vanadates et les tungstates.
de 0 à 0,01% d'agent complexant, et
un agent mouillant.

21. Procédé selon l'une quelconque des revendications précédentes, dans lequel ladite composition se présente sous forme d'une solution aqueuse.

22. Procédé de stérilisation ou de désinfection, caractérisé en ce qu'une surface devant être stérilisée ou désinfectée est mise en contact avec une composition antimicrobienne qui a été produite par le procédé selon l'une quelconque des revendications 1 à 21.
